# EUROPEAN PATENT SPECIFICATION

(11) **EP 3 033 095 B1**
(45) Date of publication and mention of the grant of the patent: **28.02.2018**
(21) Application number: 14756085.8
(22) Date of filing: 13.08.2014
(51) Int. Cl.: A61K 38/18, A61K 38/17, A61P 7/00, A61P 7/10

(54) **THERAPEUTIC USE OF VEGF-C AND CCBE1**
MEDIZINISCHE VERWENDUNG VON VEGF-C UND CCBE1
UTLISATION THÉRAPEUTIQUE DE VEGF-C ET CCBE1

(30) Priority: 14.08.2013 FI 20135832
(43) Date of publication of application: 22.06.2016
(73) Proprietor: Laurantis Pharma OY, 20520 Turku (FI)
(72) Inventor: ALITALO, Kari, FI-00014 Helsinki (FI); JELTSCH, Michael, FI-00014 Helsinki (FI); ANISIMOV, Andrey, FI-00014 Helsinki (FI)
(74) Representative: Kolster Oy Ab
(86) International application number: PCT/FI2014/050620
(87) International publication number: WO 2015/022447

(56) References cited:
- WO-A1-2010/056123
- WO-A2-2004/009773
- US-A1- 2007 110 744
- F. L. BOS ET AL: "CCBE1 Is Essential for Mammalian Lymphatic Vascular Development and Enhances the Lymphangiogenic Effect of Vascular Endothelial Growth Factor-C In Vivo", CIRCULATION RESEARCH, vol. 109, no. 5, 19 August 2011 (2011-08-19), pages 486-491, XP055149083, ISSN: 0009-7330, DOI: 10.1161/CIRCRESAHA.111.250738 cited in the application
- M. JELTSCH ET AL: "CCBE1 Enhances Lymphangiogenesis via A Disintegrin and Metalloprotease With Thrombospondin Motifs-3-Mediated Vascular Endothelial Growth Factor-C Activation", CIRCULATION, vol. 129, no. 19, 13 May 2014 (2014-05-13) , pages 1962-1971, XP055149009, ISSN: 0009-7322, DOI: 10.1161/CIRCULATIONAHA.113.002779
- HAEGERLING RENE ET AL: "A novel multistep mechanism for initial lymphangiogenesis in mouse embryos based on ultramicroscopy", EMBO (EUROPEAN MOLECULAR BIOLOGY ORGANIZATION) JOURNAL, vol. 32, no. 5, March 2013 (2013-03), pages 629-644, XP002731725,
- MARCHIO SERENA ET AL: "Emerging lymphae for the fountain of life", EMBO (EUROPEAN MOLECULAR BIOLOGY ORGANIZATION) JOURNAL, vol. 32, no. 5, March 2013 (2013-03), pages 609-611, XP002731726,

### SEQUENCE LISTING

<110> Laurantis Pharma Oy
<120> THERAPEUTIC USE OF VEGF-C AND CCBE1
<130> 2122047PC
<160> 10
<170> PatentIn version 3.5
<210> 1
   <211> 406
   <212> PRT
   <213> Homo sapiens
<400> 1
<210> 2
   <211> 6276
   <212> DNA
   <213> Homo sapiens
<400> 2
   cggccgcgga ggagcaggac gcttggtccg gacggagctc ggcgctggga agaagccggg 60
   agcttccctg atggtgccgc cgcctccgag ccggggagga gctgccaggg gccagctggg 120
   caggagcctg ggtccgctgc tgctgctcct ggcgttggga cacacgtgga cctacagaga 180
   ggagccggag gacggcgaca gagaaatctg ctcagagagc aaaatcgcga cgactaaata 240
   cccgtgtctg aagtcttcag gcgagctcac cacatgctac aggaaaaagt gctgcaaagg 300
   atataaattt gttcttggac aatgcatccc agaagattac gacgtttgtg ccgaggctcc 360
   ctgtgaacag cagtgcacgg acaactttgg ccgagtgctg tgtacttgtt atccgggata 420
   ccgatatgac cgggagagac accggaagcg ggagaagcca tactgtctgg atattgatga 480
   gtgtgccagc agcaatggga cgctgtgtgc ccacatctgc atcaatacct tgggcagcta 540
   ccgctgcgag tgccgggaag gctacatccg ggaagatgat gggaagacat gtaccagggg 600
   agacaaatat cccaatgaca ctggccatga gaagtctgag aacatggtga aagccggaac 660
   ttgctgtgcc acatgcaagg agttctacca gatgaagcag accgtgctgc agctgaagca 720
   aaagattgct ctgctcccca acaatgcagc tgacctgggc aagtatatca ctggtgacaa 780
   ggtgctggcc tcaaacacct accttccagg acctcctggc ctgcctgggg gccagggccc 840
   tcccggctca ccaggaccaa agggaagccc aggcttcccc ggtatgccag gccctcctgg 900
   gcagcccggc ccacggggct caatgggacc catgggacca tctcctgatc tgtcccacat 960
   taagcaaggc cggaggggcc ctgtgggtcc accaggggca ccaggaagag atggttctaa 1020
   gggggagaga ggagcgcctg ggcccagagg gtctccagga ccccctggtt ctttcgactt 1080
   cctgctactt atgctggctg acatccgcaa tgacatcact gagctgcagg aaaaggtgtt 1140
   cgggcaccgg actcactctt cagcagagga gttcccttta cctcaggaat ttcccagcta 1200
   cccagaagcc atggacctgg gctctggaga tgaccatcca agaagaactg agacaagaga 1260
   cttgagagcc cccagagact tctacccata gcacatccca acaccgtcac gccaaaggaa 1320
   gagaaagatc aactcacctg cagttaaacc atctaaagag aagaaagacc actggagacc 1380
   tagaaaacat acatttttct cttctcttct cctgacgtct ctccactcct cttcttccaa 1440
   atacgatgct attttcagag tcccctccta ggcctgcaga catgagggag tgaatgattg 1500
   atttacctgc ttctcactaa gagtccattg gggtggtttg cattgtaact tttcttttac 1560
   atcctatttt tccaggaact ttggatttaa gtactctcac agtgtcttaa atcataaatt 1620
   cttgaagtta aatttggcag agtatcaaaa gggggaaaat gacaaagtga gctctaagaa 1680
   aatgtgaggc tacttctaag atgtgtgttc acaatagacc ataactcctc tagtatcaaa 1740
   attggggctc ttcagttaaa aaggggtggg gaggacaaac gtgtcgatgt gctttggtgg 1800
   agaatttttt ccttgtgctt ctagtagact ttaaatattg tatccctttg tcaaaccttg 1860
   tttcccaaat tcaattaaag agaggagaga attgaatggc gtttagagaa gatagaaaag 1920
   aatcacagtc atatatttac tgttatatag attgccacat tctaaaattc aaatacggtg 1980
   cttaaggttt catgccatgc ttatctgtaa gtatcctatt tagggaagaa gattaaactc 2040
   tcttttcaaa aaaacaaagt gaaatgcctg gattcacatt aaaacaatgg gctctcgttt 2100
   gctataatat tttaaagctg tttaatcaac agtggagtct gctctataaa tatagattat 2160
   ttgttcaata aactggctga gcttagagag aggtgcagaa ttcctggttc tgagcaggtg 2220
   cccagaaggt accattaggt gccatgatcc aggctgaacc aatatacagt ggggctgaag 2280
   tctgcaagga ggttgctggc ttgggctgac ctcactaatg ccatcagcag cggtaggtaa 2340
   attttttctc cttgggtatt acaagttttt gtctggagcc aaccaagctt gccaccaaca 2400
   tattgagagt aatacactat tgaaagttat cttggatggg gagaaaaaaa aatagtggtt 2460
   ttccttgttt gcaaaaactt ccttcctatt ctcatttttt cttaattttc tttaatttag 2520
   tccaagttcc agttctttta ggccttctct ttgatttatt ttcccctgca tgtgagaagc 2580
   agttcagaaa aaggtctata tctccacctc ctagtgagtt agagtgtttt ctcagagcac 2640
   ctctgggtgg caaagggaag catgttcctg ccaaggtttg ctgtggattc agaagcacca 2700
   ggagcaagag accagaagga tgatctgctc ctttgtaacg ttgttgaggg ccctcttgtt 2760
   tccaatgagc agcttatagg ttactcacag tccactttct cactggacac acaaagtggc 2820
   tctttatcta cctttgcggg agattttcac tctcctgcaa atgatcgttc tcacactcat 2880
   attagctcat gttggaattt cccatcctgc catgtccttt cccatttctt tttggctttt 2940
   ttgcctccac cttttagccc acatcattta actccactac tgtgaaagct tgcttaaaga 3000
   aaatccctct tggccgggtg tggtagccca cgcctctaat cccagcactt tgggaggctg 3060
   aggcggggag atcacaaggt caggagatcg agaccagcct gaccaacatg gtgaaaccct 3120
   gtctctacta aaaatacaaa aattagctgg gcgtgttggc acacacctgt aatcccagct 3180
   actcaggagg ctgaggcagg agaattactt taacctgcgg ggggagccta gattgcgcta 3240
   ctgcactcca gcctaggcaa cagagggaga ctctgtctca ttaaaaaaaa aaaaaaaaaa 3300
   aaaaaaaggc cgggcttggt ggctcatgcc tgtaatccca gcactttggg aggccgaggc 3360
   gggcggatca cgaggtcagg agatcgagac cacagtgaaa ccctgtctct actaaaaaca 3420
   caaaaaatta gtcgggcgtg gtggtgggcg cctgtggtcc cagctgctcg ggaggctgag 3480
   gcaggagaat ggcgtgaacc tgggaggcgg agcttgcgct gagccgagat cgtgccactg 3540
   cactccagcc tgggcgacag agcgagactc tgtctcaaaa aaaaaaaaat atcccttttg 3600
   ttgtgtttga atgccacctt tcagtcttct tccttaagaa tgttaggaag gggctttgca 3660
   aacctctagt ggcaacagtc ttccttccct tcttcctacc acgttggaaa tgcaggactc 3720
   atgtcagtca gcatgtgacc gagtctttca tgaacacttt gctcctctat gtctcattta 3780
   tgcaaaattc ctggtgactc tccactaaca tttttaaagt gaaagcaaac tttcacttta 3840
   aaaaaagtga gcgagaggat cccttgagcc caggagttca aggccagtct gggcaacata 3900
   gcaagacccc atccctaaaa aaaaaaaaat aagttttatt aaatgtggca ctttccccta 3960
   gaagggcagt aaccccagga accagtctgc ctcttttcga ataaaggaga ctttttaaaa 4020
   agaaaaaatt tctttttagg gtggggtgct ctttatctag catctttcct tcctccttct 4080
   ctctgcaaaa ttcaacattc atgacagagg gccacaccaa ccacgggttt atagagagag 4140
   gaactgaaca gctgttgatt cccatctgga atatatttaa tgtttgcctg cttgtggagc 4200
   atggtattta aagggtgtgg atggatgaat gtttaaaaaa aaacacacaa aactatgcac 4260
   aagctgtatg agagatggct aggtccatct atgatttttt ttaaaagaag aattttaata 4320
   ccttttcttt gacagtgaac tgtatttaga aatatgatct ttattgtgaa atgctcataa 4380
   aatgcaacca aactgcatgt ttggccatag gctagaataa tatattaaaa gaattaatgg 4440
   aaagtagtat tgtctgggtt tcatgaaact attccagata atattctcca gcaaaactta 4500
   tggtaacctg tattttataa tgtcaatctg ctggaccaag ttaatttaga aaataatttt 4560
   taatcctcag attttcaaaa cttggactac tgcaagactg atatgtactt tttatgccca 4620
   aatcatattt ttcaaagata ggacatcata aggcatcatt atttctaatg tatcttttaa 4680
   ctagatagaa aaagctttac tgtctaaaca ggacttctgc tcaaaaccta atgggaaagc 4740
   tatttctggg gtaagatttg taacagtgct tctaggtcat ttcatgtttt atagggaaaa 4800
   agatctttgt acacctcctc aacctgttag tagaaatgtt gacttcgatt ttttgcaccc 4860
   tcttcacagg acctcataag ttgcctctgt tcttggaaag caatgggaat aaaaatatgc 4920
   tgttgaaaat agtgcatggg gccaggcaca gtggttatgc ctgtaatccc aacactttgg 4980
   gaggctgagg cgggcgcatc atgaggtcag gagatcaaga ccatcctgac caacacgtgg 5040
   tgaaacccca tctctactaa aataaataaa aaaaaaaatt agccaggcgt ggtggtgcac 5100
   gcctgtagtc tcagctactc gggaagctga gacacaagaa ttgcttgaac ccgggaggtg 5160
   gaggttgcag tgagccaaga ttgcgccact gcactccagc ctggctacag agtgagactc 5220
   cgtctcgaaa aaagaaaata gtacatggag ggcatgcatt ttagtttagg aaacccccct 5280
   cccttcccag ttccagaggg agaaattata agctatactg tgccaaaagt gagtggggct 5340
   gcagagagaa tggtgccttt tctcaccttc tgagttaatg cccgacatct gccattggat 5400
   tcattgacca aaggaagtca tttctactgt ataaactcag gagtatcata gcccaacaag 5460
   taaataataa atgttcattg gttttggaag gaaggaaagg tgagtattac cccaccaaat 5520
   accagcgaag agcaccctgg ctttggccat ctttccaagg ttcataattg ctgtgggcct 5580
   ggtgagccct gttcccaggt atgcaaggga gggcttctcc aggccagaca tttctgtagg 5640
   ttttcaggta gcaactcccc catcatattt tgggtgtcat tccccactct ctctccccac 5700
   ttctcagcct gtgccccaga aacaaccaca gcagcctcat tctgcagact taacatctgt 5760
   caaataacct tcctgaaaag gaaagttatt ggaggaatcc aggccaaaca acaagtagaa 5820
   tagagccatc taaagttatt aataatgagt tcagggaaat agatgaattt tttcctggca 5880
   tagtcaatat ttatatctca caacaagtca aaaataattt tccatttgga aaacaaaaca 5940
   caccatgact gtaattgtta aggcatataa tcatgaactt cacatgctcc ctcaacaaac 6000
   gttactgctc tagggaaatc atagaaaagg accctcttac tccccagggg atcaaagcaa 6060
   gtccccttag gaatttcatg tgtgccgtgg gtctgttttt atttcacagt gactagtcat 6120
   atactggagt agctctgttg ttcattattt gtaacaggtc catgtaacag tcaagttagc 6180
   attcaagcaa ttatggatgt gatactatga tgtacttttg ttatgattgt atatgcttaa 6240
   taacaaagtt atttttctta aaaaaaaaaa aaaaaa 6276
<210> 3
   <211> 419
   <212> PRT
   <213> Homo sapiens
<400> 3
   Met His Leu Leu Gly Phe Phe Ser Val Ala Cys Ser Leu Leu Ala Ala
   1               5                   10                  15
   Ala Leu Leu Pro Gly Pro Arg Glu Ala Pro Ala Ala Ala Ala Ala Phe
               20                  25                  30
   Glu Ser Gly Leu Asp Leu Ser Asp Ala Glu Pro Asp Ala Gly Glu Ala
           35                  40                  45
   Thr Ala Tyr Ala Ser Lys Asp Leu Glu Glu Gln Leu Arg Ser Val Ser
       50                  55                  60
   Ser Val Asp Glu Leu Met Thr Val Leu Tyr Pro Glu Tyr Trp Lys Met
   65                  70                  75                  80
   Tyr Lys Cys Gln Leu Arg Lys Gly Gly Trp Gln His Asn Arg Glu Gln
                   85                  90                  95
   Ala Asn Leu Asn Ser Arg Thr Glu Glu Thr Ile Lys Phe Ala Ala Ala
               100                 105                 110
   His Tyr Asn Thr Glu Ile Leu Lys Ser Ile Asp Asn Glu Trp Arg Lys
           115                 120                 125
   Thr Gln Cys Met Pro Arg Glu Val Cys Ile Asp Val Gly Lys Glu Phe
       130                 135                 140
   Gly Val Ala Thr Asn Thr Phe Phe Lys Pro Pro Cys Val Ser Val Tyr
   145                 150                 155                 160
   Arg Cys Gly Gly Cys Cys Asn Ser Glu Gly Leu Gln Cys Met Asn Thr
                   165                 170                 175
   Ser Thr Ser Tyr Leu Ser Lys Thr Leu Phe Glu Ile Thr Val Pro Leu
               180                 185                 190
   Ser Gln Gly Pro Lys Pro Val Thr Ile Ser Phe Ala Asn His Thr Ser
           195                 200                 205
   Cys Arg Cys Met Ser Lys Leu Asp Val Tyr Arg Gln Val His Ser Ile
       210                 215                 220
   Ile Arg Arg Ser Leu Pro Ala Thr Leu Pro Gln Cys Gln Ala Ala Asn
   225                 230                 235                 240
   Lys Thr Cys Pro Thr Asn Tyr Met Trp Asn Asn His Ile Cys Arg Cys
                   245                 250                 255
   Leu Ala Gln Glu Asp Phe Met Phe Ser Ser Asp Ala Gly Asp Asp Ser
               260                 265                 270
   Thr Asp Gly Phe His Asp Ile Cys Gly Pro Asn Lys Glu Leu Asp Glu
           275                 280                 285
   Glu Thr Cys Gln Cys Val Cys Arg Ala Gly Leu Arg Pro Ala Ser Cys
       290                 295                 300
   Gly Pro His Lys Glu Leu Asp Arg Asn Ser Cys Gln Cys Val Cys Lys
   305                 310                 315                 320
   Asn Lys Leu Phe Pro Ser Gln Cys Gly Ala Asn Arg Glu Phe Asp Glu
                   325                 330                 335
   Asn Thr Cys Gln Cys Val Cys Lys Arg Thr Cys Pro Arg Asn Gln Pro
               340                 345                 350
   Leu Asn Pro Gly Lys Cys Ala Cys Glu Cys Thr Glu Ser Pro Gln Lys
           355                 360                 365
   Cys Leu Leu Lys Gly Lys Lys Phe His His Gln Thr Cys Ser Cys Tyr
       370                 375                 380
   Arg Arg Pro Cys Thr Asn Arg Gln Lys Ala Cys Glu Pro Gly Phe Ser
   385                 390                 395                 400
   Tyr Ser Glu Glu Val Cys Arg Cys Val Pro Ser Tyr Trp Lys Arg Pro
                   405                 410                 415
   Gln Met Ser
<210> 4
   <211> 2076
   <212> DNA
   <213> Homo sapiens
<400> 4
   cggggaaggg gagggaggag ggggacgagg gctctggcgg gtttggaggg gctgaacatc      60
   gcggggtgtt ctggtgtccc ccgccccgcc tctccaaaaa gctacaccga cgcggaccgc     120
   ggcggcgtcc tccctcgccc tcgcttcacc tcgcgggctc cgaatgcggg gagctcggat     180
   gtccggtttc ctgtgaggct tttacctgac acccgccgcc tttccccggc actggctggg     240
   agggcgccct gcaaagttgg gaacgcggag ccccggaccc gctcccgccg cctccggctc     300
   gcccaggggg ggtcgccggg aggagcccgg gggagaggga ccaggagggg cccgcggcct     360
   cgcaggggcg cccgcgcccc cacccctgcc cccgccagcg gaccggtccc ccacccccgg   420
   tccttccacc atgcacttgc tgggcttctt ctctgtggcg tgttctctgc tcgccgctgc     480
   gctgctcccg ggtcctcgcg aggcgcccgc cgccgccgcc gccttcgagt ccggactcga     540
   cctctcggac gcggagcccg acgcgggcga ggccacggct tatgcaagca aagatctgga     600
   ggagcagtta cggtctgtgt ccagtgtaga tgaactcatg actgtactct acccagaata     660
   ttggaaaatg tacaagtgtc agctaaggaa aggaggctgg caacataaca gagaacaggc     720
   caacctcaac tcaaggacag aagagactat aaaatttgct gcagcacatt ataatacaga     780
   gatcttgaaa agtattgata atgagtggag aaagactcaa tgcatgccac gggaggtgtg     840
   tatagatgtg gggaaggagt ttggagtcgc gacaaacacc ttctttaaac ctccatgtgt     900
   gtccgtctac agatgtgggg gttgctgcaa tagtgagggg ctgcagtgca tgaacaccag     960
   cacgagctac ctcagcaaga cgttatttga aattacagtg cctctctctc aaggccccaa    1020
   accagtaaca atcagttttg ccaatcacac ttcctgccga tgcatgtcta aactggatgt    1080
   ttacagacaa gttcattcca ttattagacg ttccctgcca gcaacactac cacagtgtca    1140
   ggcagcgaac aagacctgcc ccaccaatta catgtggaat aatcacatct gcagatgcct    1200
   ggctcaggaa gattttatgt tttcctcgga tgctggagat gactcaacag atggattcca    1260
   tgacatctgt ggaccaaaca aggagctgga tgaagagacc tgtcagtgtg tctgcagagc    1320
   ggggcttcgg cctgccagct gtggacccca caaagaacta gacagaaact catgccagtg      1380
   tgtctgtaaa aacaaactct tccccagcca atgtggggcc aaccgagaat ttgatgaaaa      1440
   cacatgccag tgtgtatgta aaagaacctg ccccagaaat caacccctaa atcctggaaa      1500
   atgtgcctgt gaatgtacag aaagtccaca gaaatgcttg ttaaaaggaa agaagttcca      1560
   ccaccaaaca tgcagctgtt acagacggcc atgtacgaac cgccagaagg cttgtgagcc      1620
   aggattttca tatagtgaag aagtgtgtcg ttgtgtccct tcatattgga aaagaccaca      1680
   aatgagctaa gattgtactg ttttccagtt catcgatttt ctattatgga aaactgtgtt      1740
   gccacagtag aactgtctgt gaacagagag acccttgtgg gtccatgcta acaaagacaa      1800
   aagtctgtct ttcctgaacc atgtggataa ctttacagaa atggactgga gctcatctgc      1860
   aaaaggcctc ttgtaaagac tggttttctg ccaatgacca aacagccaag attttcctct      1920
   tgtgatttct ttaaaagaat gactatataa tttatttcca ctaaaaatat tgtttctgca      1980
   ttcattttta tagcaacaac aattggtaaa actcactgtg atcaatattt ttatatcatg      2040
   caaaatatgt ttaaaataaa atgaaaattg tattat                                2076
<210> 5
   <211> 30
   <212> DNA
   <213> Artificial Sequence
<220>
   <223> Primer
<400> 5
   gccgctagcg ccaccatggt gccgccgcct 30
<210> 6
   <211> 20
   <212> DNA
   <213> Artificial Sequence
<220>
   <223> Primer
<400> 6
   ggagcttggg cacaaatgtc 20
<210> 7
   <211> 20
   <212> DNA
   <213> Artificial Sequence
<220>
   <223> Primer
<400> 7
   accaggagca ccaggaagac 20
<210> 8
   <211> 80
   <212> DNA
   <213> Artificial Sequence
<220>
   <223> Primer
<400> 8
   gcctctagaa cgcgtctagg tgctgtccag gcccagcaga gggttaggga taggcttgcc       60
   tggataaaaa tttcttgggg                                                   80
<210> 9
   <211> 16
   <212> PRT
   <213> Artificial Sequence
<220>
   <223> Junctional amino acids of chimeric hVEGFR-3/EpoR (end of VEGFR-2 part)
<400> 9
   Phe Phe Ile Ile Glu Gly Ala Gln Glu Lys Thr Asn Leu Glu Gly Ser
   1               5                   10                  15
<210> 10
   <211> 44
   <212> PRT
   <213> Artificial Sequence
<220>
   <223> Junctional amino acids of chimeric hVEGFR-3/EpoR (start of mEpoR part)
<400> 10
   Leu Ile Leu Thr Leu Ser Leu Ile Leu Val Leu Ile Ser Leu Leu Leu
   1               5                   10                  15
   Thr Val Leu Ala Leu Leu Ser His Arg Arg Thr Leu Gln Gln Lys Ile
               20                  25                  30
   Trp Pro Gly Ile Pro Ser Pro Glu Ser Glu Phe Glu
           35                  40

## Description

### FIELD OF THE INVENTION

The present invention relates to compositions for use in treating disorders and conditions involving impaired lymphatic system, particularly lymphedema.

### BACKGROUND OF THE INVENTION

Lymphedema is a chronic disabling and disfiguring condition of localized lymph fluid retention and progressive swelling caused by a dysfunctional lymphatic system. It is a lifelong condition severely affecting quality of life of approximately 140 million patients worldwide.

Primary lymphedema, a rare inherited disease, generally results from abnormal development of the lymphatic vessels. The symptoms of primary lymphedema may occur at birth or later in life.

Acquired or secondary lymphedema results from damage to the lymph system. In Western countries, it is most commonly caused by lymph node dissection, surgery and/or radiation therapy, in which damage to the lymphatic system is caused during the treatment of cancer, particularly breast cancer. It has been estimated that in the U.S. approximately 110,000 breast cancer patients suffer from lymphedema due to axillary lymph node dissection and/or radiation, and nearly 15,000 new patients develop the breast-cancer-associated lymphedema each year.

Therapeutic lymphangiogenesis, regeneration of lymphatic vessels, is an attractive prospect for treating lymphedema. Despite the recent explosion of knowledge on the molecular mechanisms governing lymphangiogenesis the exact morphogenetic mechanisms of initial lymphangiogenesis remain incompletely understood.

Vascular endothelial growth factor C (VEGF-C) is the main driver of lymphangiogenesis in embryonic development and in various lymphangiogenic processes in adults (Alitalo, 2011, Nature Medicine 17: 1371-1380). VEGF-C acts by activating VEGFR-3 and - in its proteolytically processed mature form - also VEGFR-2. Deletion of the *Vegfc* gene in mice results in failure of lymphatic development due to the inability of newly differentiated lymphatic endothelial cells to migrate from the central veins to sites where the first lymphatic structures form (Karkkainen et al, 2003, Nature Immunology 5: 74-80; Hägerling et al, 2013, EMBO J 32: 629-644). This phenotype could be rescued by the application of recombinant VEGF-C (Karkkainen et al, 2003, ibid.). For the rescue, a "mature" recombinant form of VEGF-C was used, which lacked the N- and C-terminal propeptides. In cells secreting endogenous VEGF-C, these propeptides need to be proteolytically cleaved off from the central VEGF homology domain (VHD) in order for VEGF-C to reach its full signaling potential (Joukov et al, 1997, EMBO J 16: 3898-3911). VEGF-C can activate the main angiogenic receptor VEGFR-2 significantly only when both propeptides are cleaved off (Joukov et al, 1997, ibid.) and hence, the mature VEGF-C stimulates also angiogenesis.

Mutations in the VEGF-C receptor VEGFR-3 have been shown to result in hereditary lymphedema known as Milroy disease (Karkkainen et al, 2000, Nature Genetics 25: 153-159). On the other hand, the Hannekam lymphangiectasia-lymphedema syndrome is linked to mutations in the collagen- and calcium-binding EGF domains 1 (CCBE1) gene (Alders et al, 2009, Nature Genetics 41: 1272-1274), but it is unclear how the mutant CCBE1 causes the lymphatic phenotype. In any case, a genetic interaction of CCBE1 and VEGF-C has been suggested owing to the phenotype of the double heterozygous CCBE1+/-;VEGF-C+/- mice (Hägerling et al, 2013, ibid.).

WO 2010/056123 discloses a composition comprising CCBE1 or a polynucleotide encoding CCBE1 for use as a medicament for the treatment of a lymph vessel disorder, including lymphedema. However, CCBE1 is used alone, no combination with VEGF-C is disclosed.

US 2007/110744 discloses a method of treating a hereditary lymphedema comprising administering to said subject a composition comprising a lymphatic growth agent selected from VEGF-C polypeptides and VEGF-C polynucleotides. WO 2004/009773 discloses a method of activating VEGF-C/VEGF-D for the treatment of lymphedema. Neither US 2007/110744 nor US 2007/110744 mention CCBE1.

Although CCBE1 has been suggested to augment the pro-lymphangiogenic activity of VEGF-C in a corneal micropocket assay (Bos et al. 2011, Circulation Research 109: 486-491), its true role in lymphangiogenesis has remained obscure. One of the reasons is that, since the production of native full-length CCBE1 was impossible, the CCBE1 protein used in the experiments was an artificial truncated CCBE1 protein fused with an Fc domain of human IgG.

Despite much progress made in recent years in identifying molecules specifically expressed on lymphatic vessels, there is no curative treatment available for lymphedema. As current practise involves palliative care only, there is a need for improved therapies for lymphedema.

### BRIEF DESCRIPTION OF THE INVENTION

In one aspect, the present invention relates to a combination of full-length CCBE1 and VEGF-C for use in the treatment of lymphedema.

In some embodiments of the above aspects, the combination comprises CCBE1 in the form of a polypeptide comprising an amino acid sequence set forth in SEQ ID NO: 1, amino acids 35-406 of SEQ ID NO: 1, or an amino acid sequence having at least 80% amino acid sequence identity therewith.

In some other embodiments, the combination comprises CCBE1 in the form of a polynucleotide comprising a nucleic acid sequence set forth in SEQ ID NO: 2, nucleotides 71-1291 of SEQ ID NO: 2, or a nucleic acid sequence having at least 80% nucleic acid sequence identity therewith.

In some further embodiments, the combination comprises VEGF-C in the form of a polypeptide comprising an amino acid sequence selected from the group consisting of amino acids 32-419 of SEQ ID NO: 3; amino acids 32-227 covalently linked to amino acids 228-419 of SEQ ID NO: 3; amino acids 112-227 of SEQ ID NO: 3; amino acids 103-227 of SEQ ID NO: 3; and an amino acid sequence having at least 80% amino acid sequence identity therewith.

In some still further embodiments, the combination comprises VEGF-C in the form of a polynucleotide comprising a nucleic acid sequence selected from the group consisting of nucleic acids 524-1687 of SEQ ID NO: 4; nucleic acids 737-1687 of SEQ ID NO: 4; nucleic acids 764-1687 of SEQ ID NO: 4; nucleic acids 737-1111 of SEQ ID NO: 4; nucleic acids 764-1111 of SEQ ID NO: 4; and a nucleic acid sequence having at least 80% nucleic acid sequence identity therewith.

A further aspect of the present invention relates to a pharmaceutical composition comprising a combination of full-length CCBE1 and VEGF-C set forth in any of the embodiments described above.

Other aspects, specific embodiments, objects, details, and advantages of the invention are set forth in the following drawings, detailed description and examples.

### BRIEF DESCRIPTION OF THE DRAWINGS

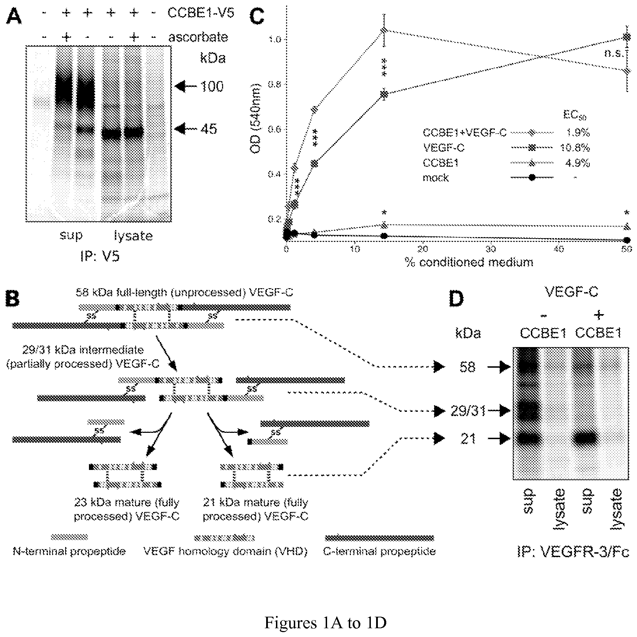

In the following the invention will be described in greater detail by means of preferred embodiments with reference to the attached drawings, in which
Figure 1 demonstrates that CCBE1 coexpression increases VEGF-C proteolytic processing to the mature, fully active VEGF-C. 293T cells were transfected with CCBE1 alone (Figure 1A) or with VEGF-C +/-CCBE1 (Figure 1D), incubated with radioactive amino acids and the medium supernatants (sup) and cell lysates were analysed by precipitation with a V5 antibody (IP: V5) and the soluble VEGF-C receptor (VEGFR-3/Fc) followed by autoradiography. Note that both intra- and extracellular CCBE1 is detected, and CCBE1 secretion is, unlike collagens, not dependent on ascorbate supplementation to the cell culture medium. Figure 1B shows a schematic view of the biosynthesis and processing of VEGF-C. Shown is the schematic structure of the VEGF-C precursor and the various processed forms (Karpanen & Alitalo, 2008, Annual Review of Pathology: Mechanisms of Disease 3: 367-397). Arrows point to the corresponding bands in panel D. Figure 1D demonstrates that VEGF-C precipitates with VEGFR-3/Fc contain uncleaved, partially cleaved and cleaved VEGF-C. Co-expression with CCBE1 decreases the amount of uncleaved VEGF-C and increases the amount of activated VEGF-C. Figure 1C illustrates that supernatants from cultures expressing both CCBE1 and VEGF-C have higher activity in promoting the growth of Ba/F3-VEGFR-3/EpoR cells than supernatants from cultures expressing only VEGF-C. Statistically significant difference of P<0.05 is marked with * and of P<0.001 with ***, n.s. indicates not statistically significant differences; n = 4.

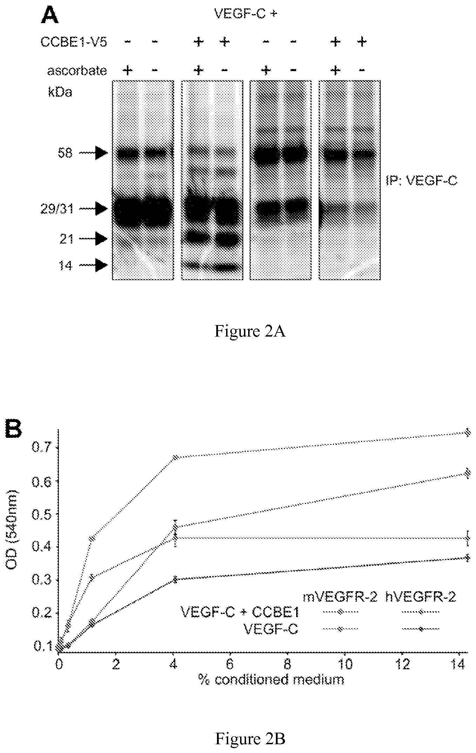

Figure 2 demonstrates that CCBE1 facilitates VEGF-C secretion. Figure 2A shows VEGF-C immunoprecipitation from supernatants and lysates of cells transfected with VEGF-C with and without CCBE1, as shown. The molecular weights on the left show the mobility of the major VEGF-C forms. Note that the amount of the mature 21 kDa form of VEGF-C increases (8-fold) in the supernatant and the amounts of the uncleaved VEGF-C and the 29/31 kDa polypeptide are reduced (by 74% and 51%, correspondingly) upon CCBE1 cotransfection (compare lanes 1, 2 to 3, 4). The 14 kDa fragment resulting from the N-terminal cleavage of VEGF-C is detected only in the supernatants of the cotransfected cells. Cotransfection with CCBE1 facilitates the secretion of VEGF-C as the intracellular VEGF-C polypeptides are reduced by 80% in the cell lysates of the CCBE1-cotransfected cells (compare lanes 5, 6 to 7, 8). Figure 2B shows that conditioned medium from cultures expressing both CCBE1 and VEGF-C have higher activity in promoting the growth of Ba/F3 cells expressing mouse (m) or human (h) VEGFR-2/EpoR chimeras than supernatants from cultures expressing only VEGF-C.

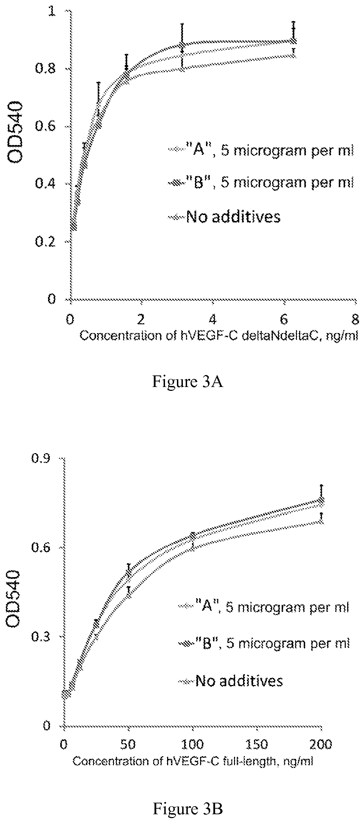

Figure 3 demonstrates that collagen-domain-truncated, Fc-fused CCBE1 does not affect the VEGFR-3 stimulating activity of VEGF-C. hVEGFR-3/EpoR-Ba/F3 cells were stimulated with increasing concentrations of human VEGF-C_{Δ}N_{Δ}C (mature VEGF-C; Figure 3A) or full-length VEGF-C (VEGF-C precursor; Figure 3B) in the presence of a constant dose (5 µg/ml) of truncated CCBE1-Fc ("A") or negative control protein, which consists only of the Fc part ("B"). Cells were incubated in stimulation conditions for a total of 3 days. At the end of this period, thiazolyl blue tetrazolium bromide (MTT, Sigma-Aldrich) was added to cell cultures and the amount of living cells in each culture (a direct score of cell proliferation) was measured by OD540 optical density (yellow MTT is reduced by mitochondrial enzymes of living cells producing dark-blue material).

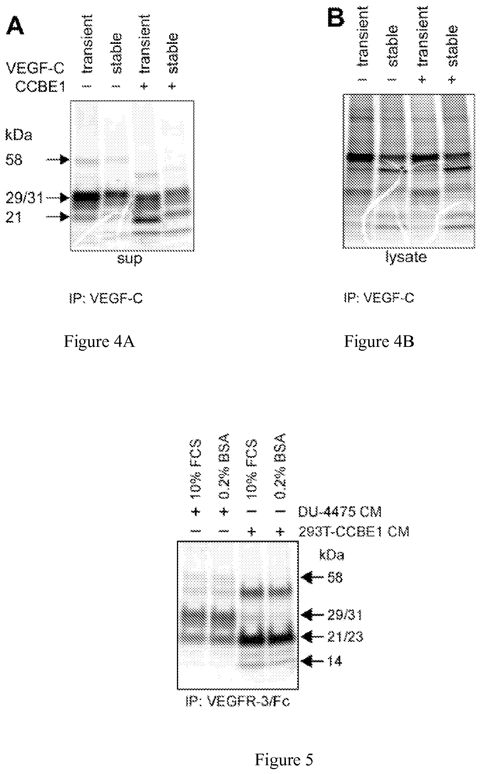

Figure 4 shows that VEGF-C cleavage is enhanced by cells expressing CCBE1 *in trans.* Separate cell populations were transfected with VEGF-C or CCBE1 and then mixed for the metabolic labeling period, as indicated. Note that when CCBE1 is (co-)transfected, the amounts of the mature VEGF-C (21 kDa) increase and the full-length (58 kDa) or partially cleaved (29/31 kDa) VEGF-C decrease in the supernatant (Figure 4A), while the amounts in the cell lysates remain unchanged (Figure 4B). Note also a small difference in the migration of VEGF-C between the stably and transiently transfected cells, resulting from the different glycosylation pattern of VEGF-C produced by the stably transfected 293S GnTI- cells (Chaudhary et al, 2012, Nature Protocols 7: 453-466).
Figure 5 shows that the VEGF-C cleavage-enhancing activity of CCBE1 is secreted into the medium, i.e. is a soluble component of the conditioned supernatant of CCBE1-producing 293T cells. This demonstrates that the activity is not restricted to the cell-surface or the extracellular matrix, but could be administered therapeutically e.g. as a soluble protein. Furthermore, the cleavage-enhancing activity is not or not significantly inhibited by the addition of 10% FCS. However, not all forms of CCBE1 do enhance the cleavage of VEGF-C. E.g. CCBE1 produced by DU-4475 doesn't enhance the cleavage of VEGF-C.

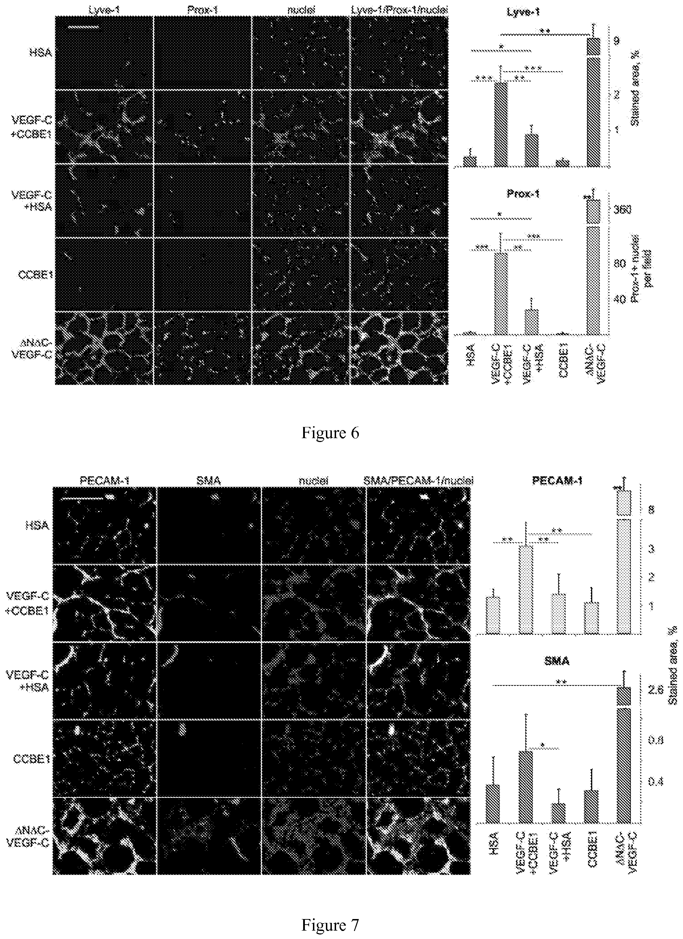

Figure 6 demonstrates that CCBE1 enhances lymphangiogenesis *in vivo.* Immunostaining of mouse tibialis anterior muscle transduced by AAV9 encoding the indicated factors and stained for the indicated antigens. Note that the full-length VEGF-C alone induces only a mild lymphangiogenic response, but its co-transduction with CCBE1 results in a strong response as detected by LYVE-1 and Prox-1 staining of lymphatic vessels. As a positive control, AAV9 encoding ΔNΔC-VEGF-C (an equivalent of the fully processed, mature VEGF-C) was used. The response to serum albumin was comparable to that of CCBE1 alone. Statistically significant difference of P<0.05 is marked with *, of P<0.01 with ** and of P<0.001 with ***, n.s. indicates that the differences are not statistically significant; n ≥ 5.
Figure 7 demonstrates that CCBE1 co-transduction with VEGF-C stimulates angiogenesis. Immunohistochemistry for endothelial (PECAM-1) and smooth muscle cell (SMA) markers in tibialis anterior muscles. Quantification of the stained areas is shown on the right. Statistically significant difference of P<0.05 is marked with *, of P<0.01 with ** and of P<0.001 with ***; n ≥ 5.

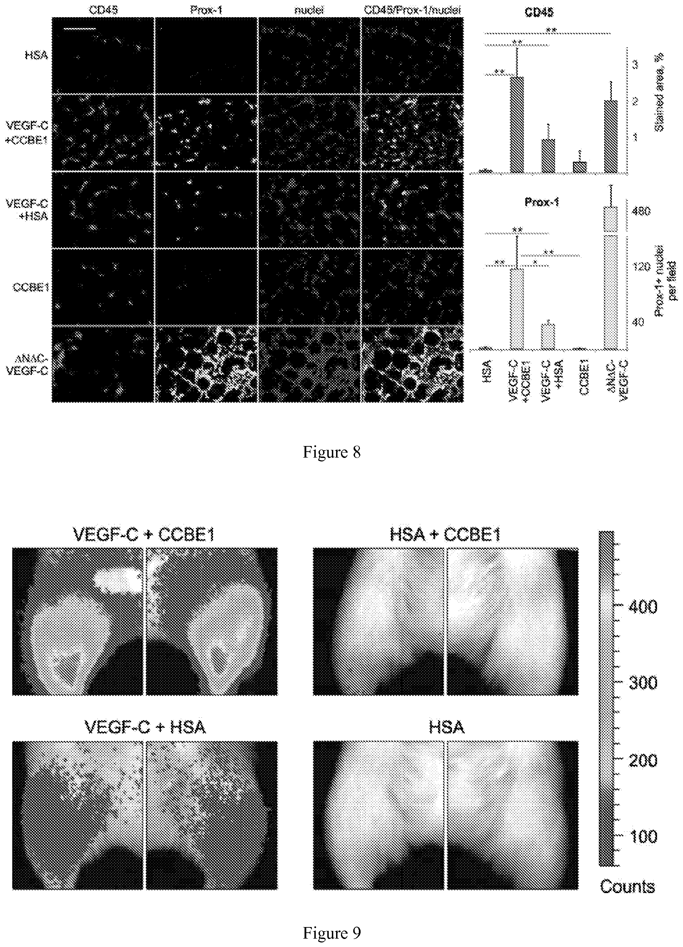

Figure 8 illustrates recruitment of CD45+ leukocytes by CCBE1/VEGF-C co-transduction. Prox1 transcription factor was used as the marker for lymphatic endothelial cells. Analysis was done as in Figure 7.
Figure 9 illustrates VEGFR-3-luciferase reporter signals in mice injected with the indicated AAV9 vectors into the tibialis anterior muscle. Note that the co-transduction with VEGF-C and CCBE1 results in a strong luciferase signal indicating a major lymphangiogenesis response, while full-length VEGF-C and CCBE1 result only in minor luciferase activity.

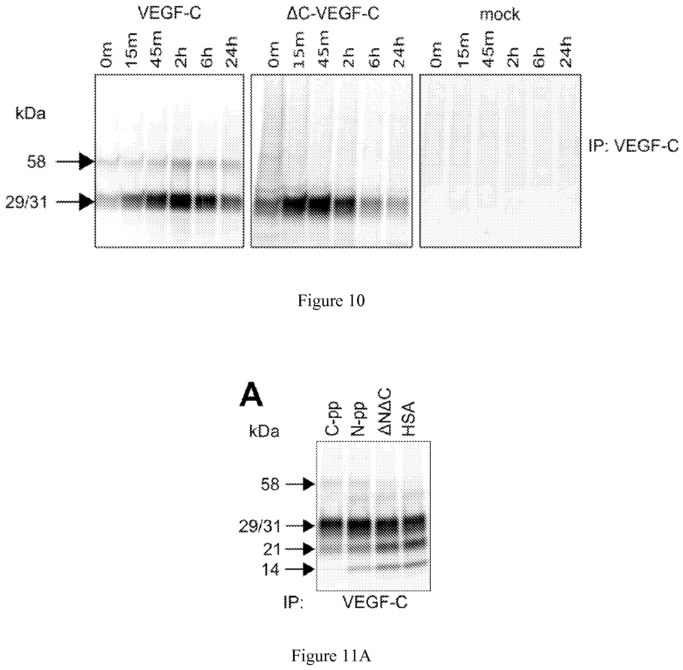

Figure 10 shows that in a pulse-chase, wild-type VEGF-C secretion peaks at 2h, whereas a VEGF-C mutant without the C-terminal propeptide (ΔC-VEGF-C) peaks already between 15 and 45 minutes.

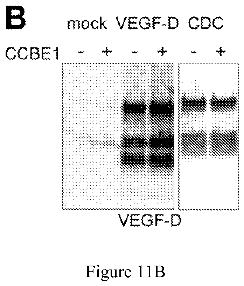

Figure 11 shows the effect of the N- and C-terminal propeptides of VEGF-C on growth factor processing. Note that the amounts of both the mature 21 kDa form of VEGF-C and the 14 kDa N-terminal propeptide are reduced by competition with the C- and N-terminal propeptides (Figure 11A). Figure 11B illustrates the proteolytic processing of VEGF-D and the VEGF-D/VEGF-C chimera (CDC) in the presence or absence of CCBE1 cotransfection. C-pp, C-terminal propeptide; N-pp, N-terminal propeptide; HSA, human serum albumin; ΔNΔC, C-and N-terminally truncated form of VEGF-C comparable to mature VEGF-C.

### DETAILED DESCRIPTION OF THE INVENTION

The invention is based on the finding that full-length collagen- and calcium-binding EGF domains 1 (CCBE1) protein promotes vascular endothelial growth factor C (VEGF-C) secretion and proteolytic cleavage of the largely inactive VEGF-C precursor, resulting in full activation of VEGF-C, which leads to increased VEGF-C receptor VEGFR-3 and VEGFR-2 signaling, lymphangiogenesis and angiogenesis *in vivo.* Thus, the present invention provides a therapeutic tool, a combination of CCBE1 and VEGF-C, for the modulation of lymphangiogenesis and angiogenesis in a variety of diseases that are associated with dysfunctional lymphatic system, such as lymphedema.

Therapeutic use of CCBE1 and VEGF-C may be implemented in various ways. For instance, co-administration of CCBE1 and VEGF-C may be achieved by gene therapy, protein therapy, or any desired combination thereof. In other words, the route and method of administration of CCBE1 and VEGF-C may be selected independently. Further, co-administration of CCBE1 and VEGF-C may be simultaneous, separate, or sequential.

As used herein, the term "or" is an inclusive "or" operator, and is equivalent to the term "and/or," unless the context clearly dictates otherwise. In addition, throughout the specification, the meaning of "a," "an," and "the" include plural references.

As used herein, the term "gene therapy" refers to the transfer of a CCBE1 or VEGF-C polynucleotide into selected target cells or tissues in a manner that enables expression thereof in a therapeutically effective amount. In accordance with the present invention, gene therapy may be used to replace a defective gene, or supplement a gene product that is not produced in a therapeutically effective amount or at a therapeutically useful time in a mammal, particularly a human, with an impaired lymphatic system.

As used herein, the term "protein therapy" refers to the administration of a CCBE1 or VEGF-C polypeptide in a therapeutically effective amount to a mammal, particularly a human, with an impaired lymphatic system for which therapy is sought. Herein, the terms "polypeptide" and "protein" are used interchangeably to refer to polymers of amino acids of any length.

As used herein, the term "therapeutically effective amount" refers to an amount of CCBE1 or VEGF-C with which the harmful effects of impaired lymphatic system are, at a minimum, ameliorated.

As used herein, the term "CCBE1" refers to a full-length CCBE1 polypeptide or to a polynucleotide encoding said full-length CCBE1, unless clearly stated otherwise. Preferably, CCBE1 is a mammalian CCBE1, preferably human. In some embodiments, the full-length CCBE1 polypeptide comprises an amino acid sequence depicted in SEQ ID NO: 1, preferably without a signal peptide. Presumably, the signal peptide of human CCBE1 consists of amino acids 1-34 of SEQ ID NO: 1 but when produced in mammalian cells, the signal peptide is automatically cleaved off correctly. It is noteworthy that the CCBE1 polypeptide, CCBE1^{Δcollagen}-Fc, disclosed by Bos et al. in Clinical Research 2011, 109: 486-491, is a truncated CCBE1 polypeptide produced from a cDNA encoding amino acids 1-191 of SEQ ID NO: 1 and the Fc portion of human IgG₁. Such a truncated CCBE1 consists of the EGF and calcium-binding EGF domains of CCBE1 fused to an Fc domain of human IgG, but lacks the collagen repeat domain.

It is evident to a person skilled in the art that the CCBE1 polypeptide to be used in accordance with the present invention may vary from the polypeptide depicted in SEQ ID NO: 1 as long as it retains its biological activity. An exemplary way of determining whether or not a CCBE1 variant has maintained its biological activity is to determine its ability to cleave full-length VEGF-C. This may be performed e.g. by incubating cells expressing full-length VEGF-C with the CCBE1 variant in question and concluding that the CCBE1 variant has retained its biological activity if VEGF-C cleavage is enhanced. Said VEGF-C cleavage may be determined e.g. by metabolic labelling and protein-specific precipitation, such as immunoprecipitation, according to methods well known in the art. If desired, CCBE1 having an amino acid sequence depicted in SEQ ID NO: 1 may be used as a positive control.

In some embodiments, the CCBE1 polypeptide comprises an amino acid sequence which is a conservative sequence variant of SEQ ID NO: 1. In connection with polypeptides, the term "conservative sequence variant" refers to amino acid sequence modifications, which arise from amino acid substitutions with similar amino acids well known in the art (e.g. amino acids of similar size and with similar charge properties) and which do not significantly alter the biological properties of the polypeptide in question. Amino acid deletions and additions are also contemplated. In some other embodiments, the CCBE1 polypeptide comprises an amino acid sequence that is at least 80%, 85%, 90%, 95%, 96%, 97%, 98%, 99% or more identical to the amino acid sequence depicted in SEQ ID NO: 1.

As used herein, the %identity between the two sequences is a function of the number of identical positions shared by the sequences (i.e. % identity = # of identical positions/total # of positions x 100), taking into account the number of gaps, and the length of each gap, which need to be introduced for optimal alignment of the two sequences. The comparison of sequences and determination of identity percentage between two sequences can be accomplished using mathematical algorithms available in the art. This applies to both amino acid and nucleic acid sequences.

As used herein, the term "CCBE1 polynucleotide" refers to any polynucleotide, such as single or double-stranded DNA or RNA, comprising a nucleic acid sequence encoding a CCBE1 polypeptide. In some embodiments, the CCBE1 polynucleotide comprises a nucleic acid sequence depicted in SEQ ID NO: 2, with 5'- and 3' untranslated regions (UTRs), or a conservative sequence variant thereof. In some preferred embodiments, the CCBE1 polynucleotide comprises a coding sequence (CDS) for full-length CCBE1, i.e. nucleic acids 71-1291 of SEQ ID NO: 2, or a conservative sequence variant thereof.

In connection with polynucleotides, the term "conservative sequence variant" refers to nucleotide sequence modifications, which do not significantly alter biological properties of the encoded polypeptide. Conservative nucleotide sequence variants include variants arising from the degeneration of the genetic code and from silent mutations. Nucleotide substitutions, deletions and additions are also contemplated. Thus, multiple CCBE1 encoding polynucleotide sequences exist for any CCBE1 polypeptide, any of which may be used therapeutically in accordance with the present invention. In some further embodiments, the CCBE1 polynucleotide may be at least 80%, 85%, 90%, 95%, 96%, 97%, 98%, 99% or more identical to the nucleic acid sequence depicted in SEQ ID NO: 2, as long as it encodes a CCBE1 polypeptide that has retained its biological activity.

As used herein, the term "VEGF-C polypeptide" refers to any known form of VEGF-C including prepro-VEGF-C, partially processed VEGF-C, and fully processed mature VEGF-C. During its biosynthesis, the full-length form of VEGF-C (58 kDa) first undergoes a proteolytic cleavage in the C-terminal part, resulting in the 29/31 kDa intermediate form held together via disulfide bonds, and a subsequent cleavage at two alternative sites in the N-terminus, yielding the mature, fully active 21 kDa or 23 kDa form of VEGF-C. This process is known to be inefficient, as the majority of VEGF-C protein does not become activated. However, the difference in the lymphangiogenic potential between the mature and the 29/31 kDa intermediate forms is remarkable (Anisimov et al, 2009, Circulation Research 104:1302-1312).

In some embodiments, the VEGF-C polypeptide to be used therapeutically in accordance with the present invention is the full-length, or prepro, form of VEGF-C. In some further non-limiting embodiments, the prepro-VEGF-C polypeptide lacks a signal sequence and, thus, may comprise amino acids 32-419 of the sequence depicted in SEQ ID NO: 3, for instance. A person skilled in the art realizes that there are alternative cleavage sites for signal peptidases and that other proteases may process the N-terminus of VEGF-C without affecting the activity thereof. Consequently, the VEGF-C polypeptide may differ from that comprising or consisting of amino acids 32-419 of SEQ ID NO: 3.

Alternatively or additionally, the VEGF-C polypeptide may be in the form of a partly processed VEGF-C, such as that comprising amino acids 32-227 covalently linked to amino acids 228-419 of the amino acid sequence depicted in SEQ ID NO: 3. Again, owing to alternative cleavage sites for signal peptidases and other proteases, the partially processed VEGF-C polypeptide may have an amino acid composition different from that of the non-limiting example described above without deviating from the present invention and its embodiments.

In some still further embodiments, the VEGF-C polypeptide to be administered to a subject suffering from impaired lymphangiogenesis is in the fully processed, or mature, form thereof such as that comprising amino acids 112-227 or 103-227 of the amino acid sequence depicted in SEQ ID NO: 3. Further, the VEGF-C polypeptide may be in any other naturally occurring or engineered form. If desired, different forms of VEGF-C polypeptides may be used in any combination. Preferably, the VEGF-C polypeptide is a mammalian VEGF-C polypeptide, more preferably human.

It is also contemplated that any of the VEGF-C polypeptides described herein may vary in their amino acid sequence as long as they retain their biological activity, particularly their capability to bind and activate VEGFR-2 and/or VEGFR-3. In some embodiments, the VEGF-C polypeptide may be a conservative sequence variant of any VEGF-C polypeptide described herein or it may comprise an amino acid sequence that is at least 85%, 90%, 95%, 96%, 97%, 98%, 99% or more identical to the amino acid sequence depicted in SEQ ID NO: 3, or any biologically relevant fragment thereof.

As used herein, the term "VEGF-C polynucleotide" refers to any polynucleotide, such as single or double-stranded DNA or RNA, comprising a nucleic acid sequence encoding any VEGF-C polypeptide. For instance, the VEGF-C polynucleotide may encode a full-length VEGF-C and comprise or consists of nucleic acids 524-1687 of a nucleic acid sequence depicted in SEQ ID NO: 4. In some other embodiments, the VEGF-C polynucleotide may encode intermediate forms of VEGF-C and comprise or consists of either nucleic acids 737-1687 or 764-1687 of the nucleic acid sequence depicted in SEQ ID NO: 4. In some further embodiments, the VEGF-C polynucleotide may encode mature forms of VEGF-C and comprise or consists of either nucleic acids 737-1111 or 764-1111 of the nucleic acid sequence depicted in SEQ ID NO: 4. None of the above embodiments contains sequences encoding a signal peptide or a stop codon but other embodiments may comprise such sequences. In some still further embodiments, the C-terminus of the mature forms may be shortened without losing receptor activation potential.

Conservative sequence variant of said nucleic acid sequences are also contemplated. Accordingly, multiple VEGF-C encoding polynucleotide sequences exist for any given VEGF-C polypeptide, any of which may be used therapeutically as described herein.

In some further embodiments, the VEGF-C polynucleotide may comprise a nucleic acid sequence which is at least 85%, 90%, 95%, 96%, 97%, 98%, 99% or more identical to the VEGF-C nucleic acid sequences described above, as long as it encodes a VEGF-C polypeptide that has retained its biological activity, particularly the capability to bind and activate VEGFR-2 and VEGFR-3.

Preferably, any CCBE1 or VEGF-C polynucleotide described herein comprises an additional N-terminal nucleotide sequence motif encoding a secretory signal peptide operably linked to the polynucleotide sequence. The secretory signal peptide, typically comprised of a chain of approximately 5 to 30 amino acids, directs the transport of the polypeptide outside the cell through the endoplasmic reticulum, and is cleaved from the secreted polypeptide. Suitable signal peptide sequences include those native for CCBE1 or VEGF-C, those derived from another secreted proteins, such as CD33, Ig kappa, or IL-3, and synthetic signal sequences.

A CCBE1 or VEGF-C polynucleotide may also comprise a suitable promoter and/or enhancer sequence for expression in the target cells, said sequence being operatively linked upstream of the coding sequence. If desired, the promoter may be an inducible promoter or a cell type specific promoter, such as an endothelial cell specific promoter. Suitable promoter and/or enhancer sequences are readily available in the art and include, but are not limited to, EF1, CMV, and CAG.

Furthermore, any CCBE1 or VEGF-C polynucleotide described herein may comprise a suitable polyadenylation sequence operably linked downstream of the coding sequence.

For gene therapy, "naked" CCBE1 or VEGF-C polynucleotides described above may be applied in the form of recombinant DNA, plasmids, or viral vectors. Delivery of naked polynucleotides may be performed by any method that physically or chemically permeabilizes the cell membrane. Such methods are available in the art and include, but are not limited to, electro-poration, gene bombardment, sonoporation, magnetofection, lipofection, liposome-mediated nucleic acid delivery, and any combination thereof.

In some other embodiments, CCBE1 or VEGF-C polynucleotides may be incorporated into a viral vector under a suitable expression control sequence. Suitable viral vectors for such gene therapy include, but are not limited to, retroviral vectors, such as lentivirus vectors, adeno-associated viral vectors, and adenoviral vectors. Preferably, the viral vector is a replication-deficient viral vector, i.e. a vector that cannot replicate in a mammalian subject. A non-limiting preferred example of such a replication-deficient vector is a replication-deficient adenovirus. Suitable viral vectors are readily available in the art.

Delivery of therapeutic CCBE1 or VEGF-C polynucleotides to a mammalian subject, preferably a human subject, may be accomplished by various ways well known in the art. For instance, viral vectors comprising CCBE1 or VEGF-C encoding polynucleotides may be administered directly into the body of the subject to be treated, e.g. by an injection into a target tissue having compromised lymphatic vessels. Such delivery results in the expression of the polypeptides *in vivo* and is, thus, often referred to as *in vivo* gene therapy. Alternatively or additionally, delivery of the present therapeutic polypeptides may be effected *ex vivo* by use of viral vectors or naked polypeptides. *Ex vivo* gene therapy means that target cells, preferably obtained from the subject to be treated, are transfected (or transduced with viruses) with the present polynucleotides *ex vivo* and then administered to the subject for therapeutic purposes. Non-limiting examples of suitable target cells for *ex vivo* gene therapy include endothelial cells, endothelial progenitor cells, smooth muscle cells, leukocytes, and especially stem cells of various kinds.

In gene therapy, expression of CCBE1 or VEGF-C may be either stable or transient. Transient expression is often preferred. A person skilled in the art knows when and how to employ either stable or transient gene therapy.

Amounts and regimens for therapeutic administration of CCBE1 and VEGF-C according to the present invention can be determined readily by those skilled in the clinical art of treating disorders or conditions, which involve impaired lymphatic vasculature. Generally, the dosage of the CCBE1 or VEGF-C treatment will vary depending on considerations such as: age, gender and general health of the patient to be treated; kind of concurrent treatment, if any; frequency of treatment and nature of the effect desired; extent of tissue damage; duration of the symptoms; and other variables to be adjusted by the individual physician. For instance, when viral vectors are to be used for gene delivery, the vector is typically administered, optionally in a pharmaceutically acceptable carrier, in an amount of 10⁷ to 10¹³ viral particles, preferably in an amount of at least 10⁹ viral particles. On the other hand, when protein therapy is to be employed, a typical dose is in the range of 0.01 to 20 mg/kg, more preferably in the range of 0.1 to 10 mg/kg, most preferably 0.5 to 5 mg/kg. A desired dosage can be administered in one or more doses at suitable intervals to obtain the desired results. A typical non-limiting daily dose may vary from about 50 mg/day to about 300 mg/day.

For protein therapy, CCBE1 or VEGF-C may be obtained by standard recombinant methods. A desired polynucleotide may be cloned into a suitable expression vector and expressed in a compatible host according to methods well known in the art. Examples of suitable hosts include but are not limited to bacteria (such as E. coli), yeast (such as S. cerevisiae), insect cells (such as SF9 cells), and preferably mammalian cell lines. Expression tags, such as His-tags, hemagglutinin epitopes (HA-tags) or glutathione-S-transferase epitopes (GST-tags), may be used to facilitate the purification of CCBE1 or VEGF-C. If expression tags are to be utilized, they have to be cleaved off prior to administration to a subject in need thereof.

As set forth above, the present therapeutic methods and uses relate to the treatment of disorders and conditions that involve impaired lymphatic vasculature. Non-limiting examples of such disorders and conditions include lymphedema, such as primary lymphedema (e.g. Milroy's syndrome, Meige's lymphedema, and lymphedema tarda), secondary lymphedema, and lipedema.

As used herein, the term "treatment" or "treating" refers to co-administration of CCBE1 and VEGF-C to a mammalian, preferably human, subject for purposes which include not only complete cure but also prophylaxis, amelioration, or alleviation of disorders or symptoms related to impaired lymphatic system. Therapeutic effect of a co-administration of CCBE1 and VEGF-C may be assessed by monitoring symptoms such as swelling, aching, discomfort, heaviness, or tightness of the affected tissue.

The present invention provides not only therapeutic methods and uses for treating disorders and conditions related to impaired lymphatic vasculature but also to pharmaceutical compositions for use in said methods and therapeutic uses. Such pharmaceutical compositions comprise CCBE1 and VEGF-C, either separately or in combination, and any pharmaceutically acceptable vehicle, such as a pharmaceutically acceptable solvent, diluent, adjuvant, excipient or carrier. For instance, the pharmaceutically acceptable vehicle may be a sterile non-aqueous carrier such as propylene glycol, polyethylene glycol, or injectable organic ester. Suitable aqueous carriers include, but are not limited to, water, saline, phosphate buffered saline, and Ringer's dextrose solution.

A variety of administration routes may be used to achieve an effective dosage to the desired site of action as well known in the art. Thus, suitable routes of administration for include, but are not limited to, parenteral delivery (e.g. intravenous injection), enteral delivery (e.g. orally), local administration, topical administration (.e.g. dermally or transdermally), as known to a person skilled in the art.

The pharmaceutical composition may be provided in a concentrated form or in a form of a powder to be reconstituted on demand. In case of lyophilizing, certain cryoprotectants are preferred, including polymers (povidones, polyethylene glycol, dextran), sugars (sucrose, glucose, lactose), amino acids (glycine, arginine, glutamic acid) and albumin. If solution for reconstitution is added to the packaging, it may consist e.g. of sterile water, sodium chloride solution, or dextrose or glucose solutions.

Means and methods for formulating the present pharmaceutical preparations are known to persons skilled in the art, and may be manufactured in a manner which is in itself known, for example, by means of conventional mixing, granulating, dissolving, lyophilizing or similar processes.

Any of the embodiments and features described above may apply independently to CCBE1 and VEGF-C and may be used in any desired combination. Thus, either one or both of CCBE1 and VEGF-C may be delivered by gene therapy or protein therapy. It is also contemplated that CCBE1 or VEGF-C may be administered using both gene therapy and protein therapy.

It will be obvious to a person skilled in the art that, as technology advances, the inventive concept can be implemented in various ways. The invention and its embodiments are not limited to the examples described below but may vary within the scope of the claims.

### EXAMPLES

### Materials and methods

**Cloning**. The genes expressed via the recombinant adeno-associated virus (rAAV9) vector were cloned into the psubCAG-WPRE plasmid (Paterna et al, 2000, Gene Therapy 7: 1304-1311), which is a derivative of psubCMV-WPRE, where the CMV promoter has been replaced with the composite CAG promoter, consisting of the chicken β-action promoter, cytomegalovirus enhancer and β-action intron (Okabe et al, 1997, FEBS Letters 407: 313-319). Cloning of full-length mVEGF-C, ΔNΔC-mVEGF-C and HSA into AAV-vector (psubCAG-WPRE) was described earlier (Anisimov et al, 2009, ibid.). mCCBE1, fused to a V5 tag (mCCBE1-V5), was cloned as follows: A partial CCBE1 coding DNA sequence (CDS; Genebank #BC152322, Image clone ID 40140631) was cloned as a Sacl/Xbal fragment into pVK1 (a pUC19-derived vector (Anisimov et al, 2007, Molecular Breeding 19: 241-253)). The missing nucleotides were amplified from brown adipose tissue mRNA with primers 5'-GCCGCTAGCGCCACCATGGTGCCGCCGCCT-3' (SEQ ID NO: 5) and 5'-GGAGCTTGGGCACAAATGTC-3' (SEQ ID NO: 6) and above CDS was completed by inserting the Nhel/Sacl fragment resulting in vector pVK1-CCBE1. A PCR-amplified V5-tag (obtained with primers 5'-ACCAGGAGCACCAGGAAGAC-3' (SEQ ID NO: 7) and 5'-GCCTCTAGAACGCGTCTAGGTGCTGTCCAGGCCCAG-CAGAGGGTTAGGGATAGGCTTGCCTGGATAAAAATTTCTTGGGG-3' (SEQ ID NO:8)) was added to the CDS as an Eco811/Xbal fragment. From the resulting vector, the complete CDS was excised as an Mlul fragment and cloned into psubCAG-WPRE.

For the in-vitro studies, an identical vector was constructed, in which the mouse CCBE1 CDS was replaced by the human CCBE1 CDS (Genebank #NM_133459), and for the co-immunoprecipitation study, a StrepIII-tag (Junttila et al, 2005, PROTEOMICS 5: 1199-1203) was inserted immediately following the CCBE1 CDS. The mammalian expression constructs for VEGF-C and ΔC-VEGF-C construct have been described before (Joukov et al, 1997, ibid.). The chimeric VEGF-C/VEGF-D (CDC) expression construct was assembled by overlapping PCR into the pMosaic vector (Jeltsch et al, 2006, J. Biol. Chem. 281: 12187-12195). The insert comprised sequences coding for amino acid residues Phe32-Ala111 and Ser228-Ser419 from human VEGF-C and intervening residues Thr92-Arg206 from human VEGF-D.

The constructs for recombinant protein expression in S2 cells employed the pMT-Ex vector (a modified version of pMT-BiP-V5His-C (Kärpänen et al, 2006, The FASEB Journal 20: 1462-1472)) and comprised sequences coding for amino acid residues Phe32-Ala111 for the N-terminal propeptide, Ser228-Ser419 for the C-terminal propeptide and Thr112-Arg227 for the ΔNΔC form of VEGF-C, followed by sequences coding for a hexahistidine tag.

**rAAV production**. rAAV9 viruses were made by a three-plasmid transfection method and purified by ultracentrifugation using discontinuous iodixanol gradient, as described (Anisimov et al, 2009, ibid.). To generate the AAV9 serotype, we used serotype-determining helper plasmid p5E18-VP2/9, instead of p5E18-VP2/8 (Michelfelder et al, 2011, PLoS ONE 6: e23101).

**Protein expression and purification.** S2 cells were transfected using Effectene (Qiagen, Venlo, The Netherlands). Stable cell pools were selected for 3 weeks with 400 µg/ml hygromycin starting 2 days after transfection. For protein production, the cells were adapted to suspension culture and induced for 4-5 days with 1 mM CuSO₄. After batch-binding to Ni²⁺NTA sepharose from the pH-adjusted conditioned supernatant, the Ni²⁺NTA sepharose was loaded onto a column, washed with 20 mM imidazole, and eluted with a step-gradient of 250 mM imidazole. The protein was further size-separated on a Superdex 200 column with PBS as a running buffer.

**Cell culture.** 293T, 293S GnTI- and NIH-3T3 cells were grown in D-MEM 10% FCS. PC-3 cells were grown in Ham's F-12 10% FCS, DU-4475 cells in RPMI 1640 20% FCS and S2 cells in HyClone SFX-Insect (Thermo-Scientific, Rockford, IL) or Insect-Xpress (Lonza Group, Basel, Switzerland).

**Transfections, metabolic labeling and protein analysis.** 293T and 293S GnTI- cells were (co)transfected with expression constructs coding for the indicated proteins. 24 hours after the transfection, the cells were metabolically labeled with [³⁵S]-cysteine/[³⁵S]-methionine (PerkinElmer, Waltham, MA) and 48 hours later, conditioned cell culture medium and cell lysates were harvested. For the short labeling experiments, harvesting was performed already after 24 hours. Alternatively, in order to produce unlabeled protein for Western blotting, the culture medium of the cells was exchanged against D-MEM 0.2% BSA and the supernatant and cell lysates were harvested 48 hours later.

Before immunoprecipitation, the samples were optionally precleared with Protein A sepharose (polyclonal antibodies and rabbit antisera) or Protein G sepharose (monoclonal antibodies). Anti-VEGF-C antiserum (Baluk et al, 2005, J. Clin. Invest. 115: 247-257), anti-V5 antibody (Invitrogen, Carlsbad, CA, #46-0705), anti-hCCBE1 antibody (Atlas Antibodies AB, Stockholm, Sweden, #HPA041374), anti-VEGF-D antibody VD1 (Achen et al, 2000, Eur. J. Biochem. 267: 2505-2515) or chimeric VEGFR-3/IgGFc (Mäkinen et al, 2001, Nature Medicine 7: 199-205) were used for immunoprecipitation.

Samples were washed and resolved by 4-20% SDS-PAGE. For autoradiography, gels were dried and exposed to phosphoimager plates or X-ray film. For the immunodetection, proteins were transferred to nitrocellulose. Specific signals were detected with anti-VEGF-C antiserum, anti-V5 antibody or anti-hCCBE1 antibody in combination with ECL. Quantitation of the autoradiography and Western blots was performed from the laser scanner read-outs or scanned X-ray film using the ImageJ software (NIH, Bethesda, MD).

**Ba/F3-VEGFR/EpoR assays**. The Ba/F3-hVEGFR-3/EpoR (Achen et al, 2000, ibid.) and Ba/F3-mVEGFR-2/EpoR (Stacker et al, 1999b) bioassays were performed with conditioned cell culture medium essentially as described (Mäkinen et al, 2001, ibid.). The Ba/F3-hVEGFR-2/EpoR cell line was generated similar as the cell lines above; however, pCl-neo was used instead of pEF-BOS. The junctional amino acid sequences of the chimera were ...FFIIEGAQEKTNLEGS (end of VEGFR-2 part) - (start of mEpoR part) LILT-LSLILVLISLLLTVLALLSHRRTLQQKIWPGIPSPESEFE... (SEQ ID NOs: 9 and 10, respectively) This chimeric construct was electroporated with one 30 ms 1400V pulse (Neon transfection device, Invitrogen) into Ba/F3 cells. Cells were grown in medium containing 2 ng/ml mIL-3 for 36 hours, after which they were split and selection was started for three weeks with 1.2 mg/ml G418. Cells were maintained with sub-optimal mIL-3 concentration (0.4 ng/ml) and optimal VEGF-A and VEGF-C concentrations (200 and 300 ng/ml, respectively).

**Pulse chase.** 293T cells were transfected and grown for 36 hours on 6-cm dishes to near confluency. Cells were starved for 30 min in met-/cys-deficient D-MEM, 5% dialyzed FCS, after which cells were metabolically labeled for 2 hours with [³⁵S]-cysteine/[³⁵S]-methionine. Thereafter, cells were washed with warm PBS and 5 ml of chase medium was added (D-MEM, 10% FCS + 2 mM cold L-methionine + 2mM cold L-Cysteine). At the indicated time points, the dishes were placed on ice and the medium was removed for analysis.

**Competition of VEGF-C cleavage by VEGF-C propeptides.** Purified histidine-tagged proteins were included in the labeling medium of the VEGF-C/CCBE1-cotransfected 293T cells at a concentration of 25 µg/ml. The labeling medium was conditioned from 30-72 hours after transfection, depleted from histidine-tagged proteins with Ni²⁺NTA sepharose, and VEGF-C was immunoprecipitated, separated by PAGE and visualized by exposure to X-ray film. Inhibition of the N-terminal cleavage of VEGF-C was measured by quantitating the 14 kDa N-terminal cleavage product from the laser-scanner read-out.

**Co-immunoprecipitation analysis of strep-tagged CCBE1.** 293T cells were transfected with either CCBE1-strepIII or full-length VEGF-C constructs. Conditioned media were used in a Strep-Tactin (Qiagen) pull-down either separately or as a mix of CCBE1 and full-length VEGF-C. Mixed media were incubated for 10 min at RT before being applied to the pull-down. Precipitates were analyzed with anti-CCBE1 antibody and anti-VEGF-C antiserum after SDS-PAGE and Western blotting. Input represents 25 µl of full-length VEGF-C conditioned medium, which was loaded as a positive control.

***In vivo* experiments.** Tibialis anterior muscles of FVB/N male mice were injected with 1:1 mixed solutions of rAAV9s encoding m(mouse)CCBE1-V5, full-length mVEGF-C or HSA. The AAV9-HSA and AAV9-ΔNΔC-mVEGF-C single vectors were used as negative and positive controls, correspondingly. Total concentration of the vector particles in a single injected dose was 6x10¹⁰. Three weeks after transduction the mice were sacrificed by CO₂ overdose. The tibialis anterior muscles were isolated, embedded into O.C.T. (Sakura Finetek Europe, Alphen aan den Rijn, The Netherlands), sectioned (10 µm thickness) and stained for the lymphatic (LYVE-1, Prox-1) and blood vascular (PECAM-1) as well as smooth muscle cell/pericyte (smooth muscle actin, SMA) and leukocyte markers (CD45), followed by Alexa-conjugated secondary antibodies (Molecular Probes, Invitrogen). Fluorescent images were obtained in an Axioplan 2 microscope (Carl Zeiss AG, Oberkochen, Germany); the objectives were: 10x NA=0.3 WD 5.6 and 20x NA=0.5 WD 2.0; the camera was a Zeiss AxioCam-HRm 14-bit greyscale CCD; the acquisition software was Zeiss AxioVision 4.6. Quantification of the stained areas was done using ImageJ software. Prox-1 positive nuclei were counted manually. The detection of luciferase activity in EGFP/Luc Vegfr3EGFP/Luc mice was performed as previously described (Martinez-Corral et al, 2012, PNAS 109: 6223-6228). The National Board for Animal Experiments of the Provincial State Office of Southern Finland approved all animal experiments carried out in this study.

**Statistical analysis.** Significance of the differences was determined using one way-ANOVA. When equal variances were assumed, Tukey's test was used as a post-hoc test. When variances were not assumed as equal, Games-Howell test was used as a post-hoc test. The EC50 of the Ba/F3 assays were calculated using logistic regression. Error bars in figures denote the standard deviation.

### Results

**CCBE1 enhances VEGF-C processing and secretion, resulting in increased VEGFR-3 activation.** CCBE1 was detected as a protein of 40-55 kDa molecular weight in both cell lysates and conditioned media of 293T cells transfected with a CCBE1 expression vector (Figure 1A). In the supernatant, part of the CCBE1 migrated as a diffuse, glycosylated band corresponding to a putative CCBE1 dimer. Transfected VEGF-C was expressed as the uncleaved 58 kDa precursor, C-terminally processed 29/31 kDa and fully processed 21 kDa mature form (Figure 1D, lane 1). However, when VEGF-C and CCBE1 were cotransfected, the amounts of the uncleaved VEGF-C and the 29/31 kDa polypeptide were reduced dramatically and the mature, fully activated VEGF-C became the major species (Figure 1D, compare lanes 1 to 3). These results indicated that CCBE1 accelerates both the N-terminal and the C-terminal proteolytic processing of VEGF-C.

In order to be able to analyze the effect of CCBE1 cotransfection on the intracellular VEGF-C forms, we used a shorter labeling period. Cotransfection with CCBE1 facilitated the secretion of VEGF-C as the intracellular amount was reduced by 80% in the lysates of the cotransfected cells (Figure 2A, compare lanes 5, 6 to 7, 8).

The conditioned medium from the CCBE1/VEGF-C cotransfected cultures stimulated the growth and survival of Ba/F3-VEGFR-3/EpoR and Ba/F3-VEGFR-2/EpoR cells better than the supernatant of cells transfected with VEGF-C alone, while CCBE1 alone showed very little activity. This confirmed that the enhanced secretion and cleavage resulted in increased levels of active VEGF-C protein in the cultures (Figure 1C and Figure 2B). Notably, also the supernatants of cells expressing CCBE1 alone resulted in a slight increase in the survival of Ba/F3-VEGFR-3/EpoR cells, presumably because of increased processing and activation of endogenous VEGF-C made by the cells.

**Truncated CCBE1 does not enhance VEGF-C stimulated VEGFR-3 activity.** CCBE1 was truncated as disclosed by Bos et al. (2011, ibid.), i.e. so that collagen-binding domain was removed and the rest of the CCBE1 molecule was fused to Fc domain of human IgG for better protein stability. The truncated CCEB1 was purified to homogeneity and tested in the established *in vitro* system together with purified full-length or mature VEGF-C protein. We run the experiment as follows. hVEGFR-3/EpoR-Ba/F3 cells were stimulated by increasing concentrations of human VEGF-CΔNΔC (mature VEGF-C) (Figure 3A) or full-length VEGF-C (VEGF-C precursor) (Figure 3B) in presence of constant dose (5 µg/ml) of truncated CCBE1-Fc or negative control protein, which is Fc part. Cells were incubated in stimulation conditions for a total of 3 days. At the end of this period, thiazolyl blue tetrazolium bromide (MTT, Sigma-Aldrich) was added to cell cultures and the numbers of alive cells in each culture (a direct score of cell proliferation) was measured by OD540 optical density (yellow MTT is reduced by mitochondrial enzymes of alive cells producing dark-blue material). The results demonstrate clearly that the truncated CCBE1 is not able to enhance the FGFR-3 stimulation activity of VEGF-C.

**CCBE1 can enhance VEGF-C processing *in trans.*** In the developing mouse embryo and zebrafish, CCBE1 is expressed adjacent to developing lymphatic structures, but not by the endothelium itself (Hogan et al, 2009, Nature Genetics 41: 396-398). Thus, we wanted to determine if CCBE1 production in *trans* by other cells can also enhance VEGF-C processing. We transfected separate cultures of 293T cells with VEGF-C or CCBE1, and mixed the cell populations 24 hours after transfection. Alternatively, we mixed CCBE1-transfected cells with cells stably expressing VEGF-C. As in the cotransfection experiments, CCBE1 increased the efficiency of the extracellular processing of VEGF-C (Figure 4A). In contrast, the intracellular processing and secretion of VEGF-C was not affected when adjacent cells produced an excess of CCBE1 (Figure 4B).

**CCBE1 enhances the lymphangiogenic activity of VEGF-C** ***in vivo**.* In order to investigate if CCBE1 enhances lymphangiogenesis *in vivo,* we transduced mouse tibialis anterior muscles with adeno-associated virus (AAV) vectors expressing CCBE1 (AAV9-CCBE1) alone, together with an AAV9-VEGF-C vector in a 1:1 ratio, or AAV9-human serum albumin (HSA) as a negative control. AAV9 encoding an activated form of VEGF-C (ΔNΔC-VEGF-C) was used as a positive control to mimic the fully proteolytically processed (mature) form of VEGF-C.

Two weeks after the AAV transduction, the skeletal muscles were analyzed by immunohistochemistry using markers for endothelial cells (PECAM-1), lymphatic endothelial cells (LYVE-1, Prox1) and leukocytes (CD45). In this assay, both full-length VEGF-C and ΔNΔC-VEGF-C stimulated lymphangiogenesis, although the latter ("mature" form) gave a considerably stronger response at the same viral dose, whereas only ΔNΔC-VEGF-C stimulated angiogenesis (Figure 6 and Figure 7, bottom row). This suggested that the proteolytic processing of full-length VEGF-C was inefficient in the AAV9 transduced muscle.

However, when full-length VEGF-C was co-transduced with CCBE1, lymphangiogenesis was significantly enhanced, as shown by the LYVE-1 and Prox-1 staining (Figure 6). We also observed significantly more angiogenesis (Figure 7), indicating that CCBE1 enhances the proteolytic processing of VEGF-C also *in vivo.* An increased number of CD45+ leukocytes was observed after co-transduction of full-length VEGF-C/CCBE1 (Figure 8), suggesting that VEGF-C activation increases also the leukocyte recruitment via VEGFR-3.

To corroborate these findings, we used AAV9-transduced heterozygous Vegfr3EGFP/Luc mice (Martinez-Corral et al, 2012, ibid.) to monitor lymphangiogenesis by optical bioluminescent imaging *in vivo.* We detected strong luciferase signals in mice co-transduced with the AAVs encoding VEGF-C and CCBE1, whereas weaker signals were detected in mice transduced with VEGF-C or CCBE1 alone, and no bioluminescent signals in mice transduced with HSA (Figure 9).

**VEGF-C propeptides are involved in CCBE1 mediated proteolysis.** Our attempts to demonstrate a physical interaction of VEGF-C and CCBE1 were unsuccessful. We thus assumed that the CCBE1-VEGF-C interaction is weak, short-lived and perhaps indirect. We tried to identify the structural element in VEGF-C that is responsible for the CCBE1-mediated accelerated secretion and cleavage. When we compared the secretion kinetics between wild-type VEGF-C and a VEGF-C mutant devoid of its C-terminal propeptide in a pulse-chase experiment, the labeled VEGF-C mutant reached its secretion peak already between 15 and 45 minutes, whereas wild-type VEGF-C reached its secretion peak only after two hours (Figure 10). This suggested that the C-terminal propeptide regulates the secretion efficiency. We tried to inhibit the CCBE1-enhanced processing of VEGF-C by adding high concentrations of the purified VEGF-C N-terminal or C-terminal propeptide or VHD. Approximately 79% inhibition of VEGF-C processing was observed with the C-terminal propeptide and about 43% inhibition with the N-terminal propeptide, while the VHD of or the BSA control did not alter the ratios of the proteolytic fragments produced by the transfected cells (Figure 11 A), confirming that the VEGF-C propeptides are involved in the CCBE1 mediated cleavages. Although VEGF-D is very similar to VEGF-C in structure and proteolytic processing (Leppänen et al, 2011, Blood 117: 1507-1515; Stacker et al, 1999a, J. Biol. Chem. 274: 32127-32136), CCBE1 cotransfection did not accelerate the proteolytic processing of human full-length VEGF-D or a chimeric factor where the VEGF-D VHD is flanked by the N- and C-terminal propeptides of VEGF-C (Figure 11B, lanes 3-6).

## Claims

1. A combination of full-length CCBE1 and VEGF-C for use in the treatment of lymphedema.

2. The combination for use according to claim 1, wherein the CCBE1 is in the form of a polypeptide comprising an amino acid sequence set forth in SEQ ID NO: 1, amino acids 35-406 set forth in SEQ ID NO: 1, or an amino acid sequence having at least 80% amino acid sequence identity therewith.

3. The combination for use according to claim 1, wherein the CCBE1 is in the form of a polynucleotide comprising a nucleic acid sequence set forth in SEQ ID NO: 2 or a nucleic acid sequence having at least 80% nucleic acid sequence identity therewith.

4. The combination for use according to claim 1, wherein the VEGF-C is in the form of a polypeptide comprising an amino acid sequence selected from the group consisting of amino acids 32-419 of SEQ ID NO: 3; amino acids 32-227 covalently linked to amino acids 228-419 of SEQ ID NO: 3; amino acids 112-227 of SEQ ID NO: 3; and amino acids 103-227 of SEQ ID NO: 3; and an amino acid sequence having at least 80% amino acid sequence identity therewith.

5. The combination for use according to claim 1, wherein the VEGF-C is in the form of a polynucleotide comprising a nucleic acid sequence selected from the group consisting of nucleic acids 524-1687 of SEQ ID NO: 4; nucleic acids 737-1687 of SEQ ID NO: 4; nucleic acids 764-1687 of SEQ ID NO: 4; nucleic acids 737-1111 of SEQ ID NO: 4; nucleic acids 764-1111 of SEQ ID NO: 4 and a nucleic acid sequence having at least 80% nucleic acid sequence identity therewith.

6. The combination for use according claim 1, wherein lymphedema is selected from the group consisting of primary lymphedema, Milroy's syndrome, Meige's lymphedema, lymphedema tarda, secondary lymphedema, and lipedema.

7. A pharmaceutical composition comprising a combination of full-length CCBE1 and VEGF-C, and a pharmaceutically acceptable vehicle.

8. The pharmaceutical composition according to claim 7, wherein the CCBE1 is in the form of a polypeptide comprising an amino acid sequence set forth in SEQ ID NO: 1, amino acids 35-406 set forth in SEQ ID NO: 1, or an amino acid sequence having at least 80% amino acid sequence identity therewith.

9. The pharmaceutical composition according to claim 7, wherein the CCBE1 is in the form of a polynucleotide comprising a nucleic acid sequence set forth in SEQ ID NO: 2 or a nucleic acid sequence having at least 80% nucleic acid sequence identity therewith.

10. The pharmaceutical composition according to claim 7, wherein the VEGF-C is in the form of a polypeptide comprising an amino acid sequence selected from the group consisting of amino acids 32-419 of SEQ ID NO: 3; amino acids 32-227 covalently linked to amino acids 228-419 of SEQ ID NO: 3; amino acids 112-227 of SEQ ID NO: 3; and amino acids 103-227 of SEQ ID NO: 3; and an amino acid sequence having at least 80% amino acid sequence identity therewith.

11. The pharmaceutical composition according to claim 7, wherein the VEGF-C is in the form of a polynucleotide comprising a nucleic acid sequence selected from the group consisting of nucleic acids 524-1687 of SEQ ID NO: 4; nucleic acids 737-1687 of SEQ ID NO: 4; nucleic acids 764-1687 of SEQ ID NO: 4; nucleic acids 737-1111 of SEQ ID NO: 4; nucleic acids 764-1111 of SEQ ID NO: 4 and a nucleic acid sequence having at least 80% nucleic acid sequence identity therewith.

## Patentansprüche

1. Kombination von Voll-Längen-CCBE1 und -VEGF-C zur Verwendung bei der Behandlung von Lymphödem.

2. Kombination zur Verwendung nach Anspruch 1, wobei das CCBE1 in Form von einem Polypeptid vorliegt, umfassend eine Aminosäuresequenz, angegeben in SEQ ID NR.: 1, Aminosäuren 35-406, angegeben in SEQ ID NR.: 1, oder eine Aminosäuresequenz mit mindestens 80 % Aminosäuresequenzidentität damit.

3. Kombination zur Verwendung nach Anspruch 1, wobei das CCBE1 in Form von einem Polynukleotid vorliegt, umfassend eine Nukleinsäuresequenz, angegeben in SEQ ID NR.: 2, oder eine Nukleinsäuresequenz mit mindestens 80% Nukleinsäuresequenzidentität damit.

4. Kombination zur Verwendung nach Anspruch 1, wobei das VEGF-C in Form von einem Polypeptid vorliegt, umfassend eine Aminosäuresequenz, ausgewählt aus der Gruppe, bestehend aus Aminosäuren 32-419 von SEQ ID NR.: 3; Aminosäuren 32-227, kovalent gebunden an Aminosäuren 228-419 von SEQ ID NR.: 3; Aminosäuren 112-227 von SEQ ID NR.: 3; und Aminosäuren 103-227 von SEQ ID NR.: 3; und eine Aminosäuresequenz mit mindestens 80 % Aminosäuresequenzidentität damit.

5. Kombination zur Verwendung nach Anspruch 1, wobei das VEGF-C in Form von einem Polynukleotid vorliegt, umfassend eine Nukleinsäuresequenz, ausgewählt aus der Gruppe, bestehend aus Nukleinsäuren 524-1687 von SEQ ID NR.: 4; Nukleinsäuren 737-1687 von SEQ ID NR.: 4; Nukleinsäuren 764-1687 von SEQ ID NR.: 4; Nukleinsäuren 737-1111 von SEQ ID NR.: 4; Nukleinsäuren 764-1111 von SEQ ID NR.: 4 und einer Nukleinsäuresequenz mit mindestens 80 % Nukleinsäuresequenzidentität damit.

6. Kombination zur Verwendung nach Anspruch 1, wobei das Lymphödem ausgewählt ist aus der Gruppe, bestehend aus primärem Lymphödem, Milroy-Syndrom, Meige-Lymphödem, Lymphödem tarda, sekundärem Lymphödem und Lipödem.

7. Pharmazeutische Zusammensetzung, umfassend eine Kombination von Voll-Längen-CCBE1 und -VEGF-C und einem pharmazeutisch verträglichen Träger.

8. Pharmazeutische Zusammensetzung nach Anspruch 7, wobei das CCBE1 in Form von einem Polypeptid vorliegt, umfassend eine Aminosäuresequenz, angegeben in SEQ ID NR.: 1, Aminosäuren 35-406, angegeben in SEQ ID NR.: 1, oder eine Aminosäuresequenz mit mindestens 80 % Aminosäuresequenzidentität damit.

9. Pharmazeutische Zusammensetzung nach Anspruch 7, wobei das CCBE1 in Form von einem Polynukleotid vorliegt, umfassend eine Nukleinsäuresequenz, angegeben in SEQ ID NR.: 2, oder eine Nukleinsäuresequenz mit mindestens 80% Nukleinsäuresequenzidentität damit.

10. Pharmazeutische Zusammensetzung nach Anspruch 7, wobei das VEGF-C in Form von einem Polypeptid vorliegt, umfassend eine Aminosäuresequenz, ausgewählt aus der Gruppe, bestehend aus Aminosäuren 32-419 von SEQ ID NR.: 3; Aminosäuren 32-227, kovalent gebunden an Aminosäuren 228-419 von SEQ ID NR.: 3; Aminosäuren 112-227 von SEQ ID NR.: 3; und Aminosäuren 103-227 von SEQ ID NR.: 3; und einer Aminosäuresequenz mit mindestens 80 % Aminosäuresequenzidentität damit.

11. Pharmazeutische Zusammensetzung nach Anspruch 7, wobei das VEGF-C in Form von einem Polynukleotid vorliegt, umfassend eine Nukleinsäuresequenz, ausgewählt aus der Gruppe, bestehend aus Nukleinsäuren 524-1687 von SEQ ID NR.: 4; Nukleinsäuren 737-1687 von SEQ ID NR.: 4; Nukleinsäuren 764-1687 von SEQ ID NR.: 4; Nukleinsäuren 737-1111 von SEQ ID NR.: 4; Nukleinsäuren 764-1111 von SEQ ID NR.: 4 und einer Nukleinsäuresequenz mit mindestens 80 % Nukleinsäuresequenzidentität damit.

## Revendications

1. Combinaison de VEGF-C et de CCBE1 de longueur totale pour utilisation dans le traitement d'un lymphoedème.

2. Combinaison pour utilisation selon la revendication 1, dans laquelle le CCBE1 se présente sous la forme d'un polypeptide comprenant une séquence d'acides aminés exposée dans SEQ ID NO : 1, des acides aminés 35-406 exposés dans SEQ ID NO : 1, ou une séquence d'acides aminés ayant au moins 80 % d'identité de séquence d'acides aminés avec celle-ci.

3. Combinaison pour utilisation selon la revendication 1, dans laquelle le CCBE1 se présente sous la forme d'un polynucléotide comprenant une séquence d'acides nucléiques exposée dans SEQ ID NO : 2 ou une séquence d'acides nucléiques ayant au moins 80 % d'identité de séquence d'acides nucléiques avec celle-ci.

4. Combinaison pour utilisation selon la revendication 1, dans laquelle le VEGF-C se présente sous la forme d'un polypeptide comprenant une séquence d'acides aminés sélectionnés dans le groupe constitué d'acides aminés 32-419 de SEQ ID NO : 3 ; d'acides aminés 32-227 liés de manière covalente à des acides aminés 228-419 de SEQ ID NO : 3 ; d'acides aminés 112-227 de SEQ ID NO : 3 ; et d'acides aminés 103-227 de SEQ ID NO : 3 ; et d'une séquence d'acides aminés ayant au moins 80 % d'identité de séquence d'acides aminés avec celle-ci.

5. Combinaison pour utilisation selon la revendication 1, dans laquelle le VEGF-C se présente sous la forme d'un polynucléotide comprenant une séquence d'acides nucléiques sélectionnés dans le groupe constitué d'acides nucléiques 524-1687 de SEQ ID NO : 4 ; d'acides nucléiques 737-1687 de SEQ ID NO : 4 ; d'acides nucléiques 764-1687 de SEQ ID NO : 4 ; d'acides nucléiques 737-1111 de SEQ ID NO : 4 ; d'acides nucléiques 764-1111 de SEQ ID NO : 4 et d'une séquence d'acides nucléiques ayant au moins 80 % d'identité de séquence d'acides nucléiques avec celle-ci.

6. Combinaison pour utilisation selon la revendication 1, dans laquelle un lymphoedème est sélectionné dans le groupe constitué du lymphoedème primaire, du syndrome de Milroy, du lymphoedème de Meige, du lymphoedème tardif, du lymphoedème secondaire, et du lipoedème.

7. Composition pharmaceutique comprenant une combinaison de VEGF-C et de CCBE1 de longueur totale, et un véhicule pharmaceutiquement acceptable.

8. Composition pharmaceutique selon la revendication 7, dans laquelle le CCBE1 se présente sous la forme d'un polypeptide comprenant une séquence d'acides aminés exposée dans SEQ ID NO : 1, des acides aminés 35-406 exposés dans SEQ ID NO : 1, ou une séquence d'acides aminés ayant au moins 80 % d'identité de séquence d'acides aminés avec celle-ci.

9. Composition pharmaceutique selon la revendication 7, dans laquelle le CCBE1 se présente sous la forme d'un polynucléotide comprenant une séquence d'acides nucléiques exposée dans SEQ ID NO : 2 ou une séquence d'acides nucléiques ayant au moins 80 % d'identité de séquence d'acides nucléiques avec celle-ci.

10. Composition pharmaceutique selon la revendication 7, dans laquelle le VEGF-C se présente sous la forme d'un polypeptide comprenant une séquence d'acides aminés sélectionnés dans le groupe constitué d'acides aminés 32-419 de SEQ ID NO : 3 ; d'acides aminés 32-227 liés de manière covalente à des acides aminés 228-419 de SEQ ID NO : 3 ; d'acides aminés 112-227 de SEQ ID NO : 3 ; et d'acides aminés 103-227 de SEQ ID NO : 3 ; et d'une séquence d'acides aminés ayant au moins 80 % d'identité de séquence d'acides aminés avec celle-ci.

11. Composition pharmaceutique selon la revendication 7, dans laquelle le VEGF-C se présente sous la forme d'un polynucléotide comprenant une séquence d'acides nucléiques sélectionnés dans le groupe constitué d'acides nucléiques 524-1687 de SEQ ID NO : 4 ; d'acides nucléiques 737-1687 de SEQ ID NO : 4 ; d'acides nucléiques 764-1687 de SEQ ID NO ; 4 ; d'acides nucléiques 737-1111 de SEQ ID NO : 4 ; d'acides nucléiques 764-1111 de SEQ ID NO : 4 et d'une séquence d'acides nucléiques ayant au moins 80 % d'identité de séquence d'acides nucléiques avec celle-ci.
